# EUROPEAN PATENT SPECIFICATION

(11) **EP 0 433 624 B1**
(45) Date of publication and mention of the grant of the patent: **29.06.1994**
(21) Application number: 90120881.9
(22) Date of filing: 31.10.1990
(51) Int. Cl.: A61K 31/44, A61K 9/06, A61K 9/12, A61K 47/10, A61K 47/14, A61K 47/44

(54) **Pharmaceutical compositions for the topical use containing pyridinol carbamate**
Lokal anzuwendende, Pyridinolcarbamat enthaltende Arzneizubereitungen
Compositions pharmaceutiques à application topique à base de carbamate de pyridinol

(30) Priority: 07.11.1989 IT 2227989
(43) Date of publication of application: 26.06.1991
(73) Proprietor: Angeletti, Ruggero, I-00144 Rome (IT)
(72) Inventor: Angeletti, Ruggero, I-00144 Rome (IT)
(74) Representative: Minoja, Fabrizio

(56) References cited:
- US-A- 3 467 663
- DATABASE: WPI, accession no. 77-11749Y, Derwent Publications Ltd, London, GB; & JP-A-51 151 342 (SUN STAR) 26-12-1976
- DATABASE: WPIL, accession no. 85-119071, Derwent Publications Ltd, London, GB; & JP-A-60 058 918 (ADVANCE KAIHATSU) 05-04-1985
- Harry's Cosmeticology, 7th ed (1982), pp 615-616j
- Martindale, The Extra Pharmacopoeia, 28th ed. (1982) page 1749
- Comprehensive Medicinal Chemistry, Vol 1, pages 593-606

## Description

### DISCLOSURE

The present invention relates to pharmaceutical formulations for the topical use containing 2,6-pyridinemethanol-bis-(N-methylcarbamate) (pyridinol carbamate) for the treatment of obliterans peripheral vasculopathies.

Compound (I) of formula: 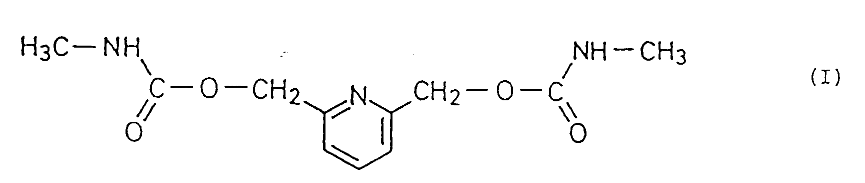
which is also known under the name of pyridinol carbamate, is used in oral pharmaceutical formulations. It can be obtained starting from pyridine-2,6-dicarboxylic acid, by means of processes which are described, inter alia, in French Patents 1.396.624 and 1.394.362.

Tooth-pastes containing pyridinol carbamate are known from JP-A-51 151 342 (Sun Star)
Up to now known formulations of pyridinol carbamate, which can be administered by the oral route, as already stated, are used as coadjuvants in the treatment of obliterans peripheral vasculopathies and of atherosclerotic retinopathies. They are generally administered in form of a 500 mg tablet, 2 or 3 times a day.

Even though oral formulations of pyridinol carbamate proved to be satisfactorily effective, they suffer from disadvantages of various origin : gastrointestinal intolerance, palpitation or tachycardia, cephalgia and vertigo frequently occur. An increase in transaminases and hepatitis also sometimes takes place, so as periodical checks of hepatic functional capacity are suggested in case of pyridinol carbamate oral treatment.

Now it has surprisingly been found that pyridinol carbamate can be administered under the form of topical formulations, which show a dramatical effectiveness against topical atherosclerosis conditions, using a posology by far lower than that hitherto employed. In practice, using the formulations of the invention, very good therapeutical results can be obtained, by administering active ingredient amounts generally ranging from about one fifth to about one tenth of the dose administered up to now. Moreover, the drug, when administered by the topical route, exerts its activity in much shorter times, reaching more easily the action site, particularly in case of phlebopathies or venous stasis ulcers. Furthermore, the frequent gastrointestinal and hepatic drawbacks, often involved in the treatment with oral formulations, are of course avoided.

Pyridinol carbamate by the topical route is suited mainly as a peripheral antiatherosclerotic agent, in phlebopathies and varicose ulcers, in decubitus ulcers and the like.

Moreover, pyridinol carbamate is used in the cosmetological field as a tonifying and elasticizing agent for the skin and the underlying connective tissue, as well as of cutaneous unaesthetisms caused by couperose and varices.

The active ingredient can be solubilized in the same way as the one used for the topical route or with liposomes, to be used for the preparation of a sterile ointment for the ophthalmic administration. The formulation according to the invention is particularly useful in the treatment of eye vascular diseases of thrombotic and hemorrhagic origin. In fact, it causes a marked improvement of visual acuity and of the ophthalmologic conditions already after the first weeks of treatment.

It may also be used for the same disease (angina pectoris) in two different ways: the first in aerosol for the attacks, the second using sticking plaster for the maintaining cure.

Pyridinol carbamate is solubilized in a base allowing topical administration, using hydrophobic solvents, such as natural and synthetic solvents or carriers.

Hydrophobic solvents or carriers are the esters of C₁-C₂₆ fatty acids with C₁-C₄ lower alcohols, such as the esters of C₁-C₄ lower alcohols with formic, acetic, propionic, butyric, valerianic, caproic, heptanoic, caprylic, pelargonic, caprinic, undecanoic, lauric, tridecanoic, myristic, pentadecanoic, palmitic, margaric, stearic, nonadecanoic, arachic, behenic acids and the like; with the corresponding branched chain acids, such as isobutyric, isovalerianic, pivalic, 2-ethylhexanoic acids; with mono- or poly-unsaturated acids, such as crotonic, undecylenic, behenic, eicosapentenoic acids. The lower esters with saturated or unsaturated straight or branched carboxylic acids, having one or more hydroxy or amino groups in their chain, can also be used. As solubilizing agents natural or synthetic glycerides can be used.

Particularly suited for the preparation of the formulations according to the invention are also the liposoluble extracts from Centella asiatica, Equisetum, Malva and the like; as well as natural waxes such as carnauba wax, candelilla wax and jojoba wax.

Particularly favourable results are obtained using lower esters of C₁₀-C₁₈ fatty acids, particularly isopropyl myristate, which solubilizes about 100% by weight of pyridinol carbamate.

The topical formulations of the invention can also be prepared starting from mixtures of the above mentioned solubilizing agent and cream bases such as Dermobase^{R} by Borden or Cold Cream^{R} by Schering, or with lanolin.

Pyridinol carbamate concentration in the compositions according to the invention can range from 1 to 30% by weight, preferably from 5 to 10% by weight.

The following examples further illustrate the invention.

### EXAMPLE 1

5 Parts by weight of pyridinol carbamate are dissolved in 10 parts by weights of Equisetum liposoluble extract, heating to about 35-40°C. The solution obtained is homogeneously mixed with Dermobase^{R} in such an amount as to reach 100 parts by weight.

### EXAMPLE 2

The procedure described in Example 1 is repeated, but replacing Equisetum liposoluble extract with an equal amount of Malva liposoluble extract.

### EXAMPLE 3

The procedure described in Example 1 is repeated, but using a Centella asiatica liposoluble extract.

### EXAMPLE 4

The procedure described in Example 1 is repeated, but increasing pyridinol carbamate percentage to 10%.

### EXAMPLE 5

5 Parts by weight of pyridinol carbamate are dissolved in 2.5 parts by weight of isopropyl myristate, heating slightly. The obtained solution is the homogeneously mixed with Cold Cream^{R} in such an amount as to reach 100 parts by weight.

### EXAMPLE 6

The procedure described in Example 5 is repeated, but adding 1% by weight of undecylenic acid.

### EXAMPLE 7

The procedure described in the preceding Examples is repeated, but adding 1.5% by weight of water.

### EXAMPLE 8

The procedure described in the preceding examples is repeated, but adding suited amounts of liposomes.

## Claims

1. A pharmaceutical composition for the topical or ophthalmic use containing pyridinol carbamate as the active ingredient dissolved in a solvent or carrier selected from esters of C₁-C₂₆ fatty acids with C₁-C₄ alcohols, liposoluble extracts from Centella asiatica, Equisetum, Malva or Carnauba, Candelilla or Jojoba wax.

2. A pharmaceutical composition as claimed in claim 1, characterized in that lower esters of C₁₀-C₁₈ fatty acids are used.

3. A pharmaceutical composition as claimed in claims 1-2, characterized in that isopropyl myristate is used.

4. The use of pyridinol carbamate for the preparation of a topical medicament for the treatment of topical atherosclerosis conditions.

## Patentansprüche

1. Pharmazeutisches Präparat zur topischen oder ophthalmologischen Verwendung, enthaltend Pyridinolcarbamat als Wirkstoff, aufgelöst in einem Lösungsmittel oder einem Träger, ausgewählt aus Estern der C₁-C₂₆-Fettsäuren mit C₁-C₄ Alkoholen, fettlöslichen Extrakten aus Centella asiatica, Equisetum, Malva oder Carnauba-, Candelilla- oder Jojobawachs.

2. Pharmazeutischen Präparat nach Anspruch 1, dadurch **gekennzeichnet**, daß niedrige Ester von C₁₀-C₁₈-Fettsäuren verwendet werden.

3. Pharmazeutisches Präparat nach Anspruch 1 bis 2, dadurch **gekennzeichnet**, daß Isopropylmyristat verwendet wird.

4. Verwendung von Pyridinolcarbamat zur Herstellung eines topischen Medikaments zur Behandlung von topischen atherosklerotischen Zuständen.

## Revendications

1. Composition pharmaceutique pour une utilisation topique ou ophtalmique, contenant du carbamate de pyridinol comme ingrédient actif dissous dans un solvant ou support choisi parmi les esters d'acides gras en C₁-C₂₆ avec des alcools en C₁-C₄, les extraits liposolubles de Centella asiatica, Equisetum, Malva ou Carnauba, la cire de Candelilla ou de Jojoba.

2. Composition pharmaceutique selon la revendication 1, caractérisée par le fait que des esters inférieurs d'acides gras en C₁₀-C₁₈ sont utilisés.

3. Composition pharmaceutique selon les revendications 1 et 2, caractérisée par le fait que du myristate d'isopropyle est utilisé.

4. Utilisation du carbamate de pyridinol pour la préparation d'un médicament topique pour le traitement d'états d'athérosclérose topique.
